# EUROPÄISCHE PATENTANMELDUNG

(11) **EP 4 732 884 A1**
(43) Veröffentlichungstag der Anmeldung: **29.04.2026**
(21) Anmeldenummer: 24208709.6
(22) Anmeldetag: 24.10.2024
(51) Int. Cl.: A61M 37/00, A61B 5/151

(54) **NADELMODUL FÜR EIN HANDGERÄT ZUM LOKALEN AUFSTECHEN EINER HAUT, HANDGERÄT SOWIE VERFAHREN ZUM BETREIBEN EINES NADELMODULS**

(71) Anmelder: MT.DERM GmbH, 12307 Berlin (DE)
(72) Erfinder: SCHERKOWSKI, Dirk, 12489 Brelin (DE)
(74) Vertreter: Bittner, Thomas L.

(57) **Zusammenfassung**

Die Erfindung betrifft ein Nadelmodul (4) für ein Handgerät zum lokalen Aufstechen einer Haut mit: einem Modulgehäuse (5), mit einer frontseitigen Modulöffnung (7); einer Stecheinrichtung mit einer an einem Nadelschaft (14) angeordneten Stechnadel die im Modulgehäuse (5) aufgenommen ist, derart, dass die Stechnadel (9) zwischen einer eingefahrenen und einer ausgefahrenen Stellung linear verlagerbar ist; und einer Übertragungseinrichtung, (i) die im Modulgehäuse (5) aufgenommen und betreibbar ist, eine antriebsseitig mit einer Wiederholfrequenz bereitgestellte Antriebsbewegung auf den Nadelschaft (14) zu übertragen, derart, dass die die w Stechnadel (9) wiederholt zwischen der eingefahrenen und der ausgefahrenen Stellung verlagert wird; und (ii) die eine Rolleinrichtung (16) aufweist, die ein erstes drehabers Rollbauteil (17) und ein zweites Rollbauteil (18) aufweist und auf die Antriebsbewegung einleitbar ist, derart, dass beim Übertragen der Antriebsbewegung das erste Rollbauteil (17) auf einer Rollfläche (21) an dem zweiten Rollbauteil (18) abrollt.

## Beschreibung

Die Erfindung betrifft ein Nadelmodul für ein Handgerät zum lokalen Aufstechen einer menschlichen oder einer tierischen Haut, ein Handgerät zum lokalen Aufstechen einer menschlichen oder einer tierischen Haut und ein Verfahren zum Betreiben eines Nadelmoduls.

### Hintergrund

Solche Nadelmodule werden bei Handgeräten zum lokalen Aufstechen einer menschlichen oder einer tierischen Haut verwendet, um eine Stecheinrichtung bereitzustellen, mit der die Haut lokal wiederholt aufgestochen werden kann. Das lokale Aufstechen der Haut kann insbesondere dazu dienen, einen Farbstoff, einen medizinischen und / oder einen kosmetischen Wirkstoff in die Haut einzubringen. Aber auch ohne Stoffeintrag in die Haut werden solche Handgeräte verwendet, um die Haut lokal zu stimulieren.

Neben dem Nadelmodul weist das Handgerät zum lokalen Aufstechen der Haut regelmäßig eine Antriebseinrichtung auf, mit der zum Betrieb der Stecheinrichtung des Nadelmoduls repetierend eine Antriebsbewegung oder Antriebskraft bereitgestellt wird, insbesondere um eine oder mehrere Stechnadeln der Stecheinrichtung zwischen einer eingefahrenen und einer ausgefahrenen Stellung zu verlagern. Auf diese Weise kann eine Nadelspitze der Stechnadel im Betrieb wiederholt lokal in die Haut eingestochen und aus der Haut zurückgezogen werden.

### Zusammenfassung

Aufgabe der Erfindung ist es, ein Nadelmodul für ein Handgerät zum lokalen Aufstechen einer menschlichen oder einer tierischen Haut, ein Handgerät zum lokalen Aufstechen einer menschlichen oder einer tierischen Haut sowie ein Verfahren zum Betreiben eines Nadelmoduls anzugeben, bei denen eine bereitgestellte Antriebsbewegung effizient und betriebssicher auf eine Stecheinrichtung übertragen werden kann.

Zur Lösung sind ein Nadelmodul für ein Handgerät zum lokalen Aufstechen einer menschlichen oder einer tierischen Haut sowie ein Handgerät zum lokalen Aufstechen einer menschlichen oder einer tierischen Haut nach den unabhängigen Ansprüchen 1 und 15 geschaffen. Weiterhin ist ein Verfahren zum Betreiben eines Nadelmoduls gemäß dem nebengeordneten Anspruch 16 geschaffen. Ausgestaltungen sind Gegenstand von abhängigen Unteransprüchen.

Nach einem Aspekt ist ein Nadelmodul für ein Handgerät zum lokalen Aufstechen einer menschlichen oder einer tierischen Haut geschaffen, welches Folgendes aufweist: ein Modulgehäuse, welches eine Modulspitze und eine im Bereich der Modulspitze ausgebildete frontseitige Modulöffnung aufweist; eine Stecheinrichtung, welche wenigstens eine an einem Nadelschaft angeordnete Stechnadel mit einer Nadelspitze aufweist und in dem Modulgehäuse aufgenommen ist, derart, dass die wenigstens eine Stechnadel mittels einer linearen Bewegung zwischen einer eingefahrenen und einer ausgefahrenen Stellung verlagerbar ist, wobei sich die wenigstens eine Stechnadel zumindest in der ausgefahrenen Stellung durch die fronseitige Modulöffnung erstreckt und die Nadelspitze hierbei außerhalb des Modulgehäuses angeordnet ist; und eine Übertragungseinrichtung. Die Übertragungseinrichtung ist wenigstens teilweise in dem Modulgehäuse aufgenommen und betreibbar, eine antriebsseitig mit einer Wiederholfrequenz bereitgestellte Antriebsbewegung auf den Nadelschaft zu übertragen, derart, dass die übertragene Antriebsbewegung abtriebsseitig auf die wenigstens eine Stechnadel einkoppelbar ist, um diese wiederholt zwischen der eingefahrenen und der ausgefahrenen Stellung zu verlagern. Die Übertragungseinrichtung weist eine Rolleinrichtung auf, die ein erstes Rollbauteil, welches drehbar gelagert ist, und ein zweites Rollbauteil aufweist und auf die die antriebsseitig bereitgestellte Antriebsbewegung einleitbar ist, derart, dass beim Übertragen der Antriebsbewegung eine Rollbewegung ausgeführt wird, bei der das erste Rollbauteil auf einer Rollfläche an dem zweiten Rollbauteil rollt.

Nach einem weiteren Aspekt ist ein Handgerät zum lokalen Aufstechen einer menschlichen oder einer tierischen Haut geschaffen, welches das vorgenannte Nadelmodul sowie eine Antriebseinrichtung aufweist, die zum Übertragen einer von der Antriebseinrichtung bereitgestellten Antriebsbewegung auf das Nadelmodul funktional an das Nadelmodul koppelt.

Nach einem weiteren Aspekt ist ein Verfahren zum Betreiben eines Nadelmoduls mit den folgenden Schritten geschaffen: Bereitstellen eines Nadelmoduls, ein Modulgehäuse, welches eine Modulspitze und eine im Bereich der Modulspitze ausgebildete frontseitige Modulöffnung aufweist; eine Stecheinrichtung, welche wenigstens eine an einem Nadelschaft angeordnete Stechnadel mit einer Nadelspitze aufweist und in dem Modulgehäuse aufgenommen ist; und eine Übertragungseinrichtung, die wenigstens teilweise in dem Modulgehäuse aufgenommen ist und eine Rolleinrichtung aufweist; antriebsseitiges Einleiten einer mit einer Wiederholfrequenz bereitgestellten Antriebsbewegung auf die Übertragungseinrichtung; Übertragen der Antriebsbewegung mittels der Übertragungseinrichtung, wobei hierbei mittels der Rolleinrichtung eine Rollbewegung ausgeführt wird, bei der ein erstes Rollbauteil der Rolleinrichtung, welches drehbar gelagert ist, auf einer Rollfläche an einem zweiten Rollbauteil der Rolleinrichtung abrollt; und abtriebsseitiges Einkoppeln der übertragenen Antriebsbewegung auf den Nadelschaft, derart, dass die wenigstens eine Stechnadel mittels einer linearen Bewegung wiederholt zwischen einer eingefahrenen und einer ausgefahrenen Stellung verlagert wird, wobei sich die wenigstens eine Stechnadel zumindest in der ausgefahrenen Stellung durch die fronseitige Modulöffnung erstreckt und die Nadelspitze hierbei außerhalb des Modulgehäuses angeordnet ist.

Die mit der Wiederholfrequenz bereitgestellte Antriebsbewegung oder -kraft wird mit Hilfe der vorgeschlagenen Übertragungseinrichtung effizient und betriebssicher auf den Nadelschaft der Stecheinrichtung und somit schließlich auf die wenigstens eine Stechnadel übertragen, um diese im Betrieb zwischen der eingefahrenen und der ausgefahrenen Stellung zu verlagern. Die repetierende Bereitstellung der Antriebsbewegung ermöglicht eine lineare repetierende Bewegung der wenigstens einen Stechnadel zwischen der eingefahrenen und der ausgefahrenen Stellung. Hierzu weist die Übertragungseinrichtung die Rolleinrichtung auf, derart, dass mittels der Rollbewegung zwischen dem ersten und dem zweiten Rollbauteil die Antriebsbewegung mit der Wiederholfrequenz übertragbar ist, wobei das drehbar gelagerte erste Rollbauteil hierbei auf der Rollfläche an dem zweiten Rollbauteil rollt. Die Rollbewegung umfasst wiederholtes Vor- und Zurückrollen des ersten Rollbauteils auf der Rollfläche an dem zweiten Rollbauteil, um so die Antriebsbewegung wiederholt oder repetierend mit der Wiederholfrequenz zu übertragen, so dass diese von der Übertragungseinrichtung abtriebsseitig auf den Nadelschaft und somit auf die wenigstens eine Stechnadel einkoppelbar ist. Die mit Hilfe der Rolleinrichtung in der Übertragungseinrichtung bereitgestellte Rollbewegung unterstützt eine möglichst geräuscharme Übertragung der Antriebsbewegung.

Bei dem Handgerät wird die Antriebsbewegung oder -kraft mittels der Antriebseinrichtung bereitgestellt, die zum Beispiel mit einem elektrischen Motor gebildet ist. Die Antriebseinrichtung kann hierbei in einem Handstück oder -modul aufgenommen sein, dessen Gehäuse ein Nutzer bei der Verwendung des Handgeräts mit den Fingern greifen kann. Das Nadelmodul kann mit dem Handstück lösbar verbunden sein, wodurch zum Beispiel eine Ausbildung des Nadelmoduls als Einwegmodul ermöglicht ist.

Die mit der Wiederholfrequenz von der Antriebseinrichtung bereitgestellte Antriebsbewegung oder -kraft kann in das Nadelmodul mit Hilfe eines Kopplungsbauteils eingekoppelt werden, zum Beispiel einer Kopplungsstange oder einem Kopplungsstift. Das Kopplungsbauteil kann sich hierbei durch eine rückseitige Modulöffnung des Modulgehäuses des Nadelmoduls erstrecken. Die rückseitige Modulöffnung kann mittels einer Dichteinrichtung verschlossen sein, so dass ein Innenraum des Modulgehäuses des Nadelmoduls rückseitig gegenüber der Umgebung abgedichtet ist, zum Beispiel mit Hilfe einer Dichtmembran. Das die Antriebsbewegung in das Nadelmodul einkoppelnde Kopplungsbauteil kann gedichtet durch eine Membran-öffnung der Dichtmembran hindurchgeführt sein.

Die Rolleinrichtung kann ein drittes Rollbauteil aufweisen, und beim Übertragen der Antriebsbewegung kann eine weitere Rollbewegung ausgeführt werden, bei der das erste Rollbauteil auf einer weiteren Rollfläche an dem dritten Rollbauteil abrollt. Bei dieser Ausführungsform führt das erste Rollbauteil mehrere Rollbewegungen an unterschiedlichen Rollflächen aus und wird so jeweils an den mehreren Rollflächen geführt.

Bei der Rolleinrichtung kann eine der folgenden Ausführungsformen vorgesehen sein: (i) das zweite und das dritte Rollbauteil sind in Bezug auf die Drehachse des ersten Rollbauteils auf gegenüberliegenden Seiten angeordnet; und (ii) das zweite und das dritte Rollbauteil sind in Bezug auf die Drehachse des ersten Rollbauteils auf der gleichen Seite benachbart zueinander angeordnet. Sind das erste und das dritte Rollbauteil auf gegenüberliegenden Seiten der Drehachse des ersten Rollbauteils angeordnet, können die am zweiten und am dritten Rollbauteil vorgesehenen Rollflächen einander gegenüberliegend angeordnet sein. Sind das zweite und das dritte Rollbauteil in Bezug auf die Drehachse des ersten Rollbauteils auf der gleichen Seite und benachbart zueinander angeordnet, können die Rollflächen an dem zweiten und an dem dritten Rollbauteil nebeneinanderliegen, sei es in vertikaler oder in horizontaler Richtung, sich in Bewegungsrichtung der Rollbewegung insbesondere parallel zueinander erstrecken.

Bei der Rolleinrichtung kann eine der folgenden Ausführungsformen vorgesehen sein: (i) das erste Rollbauteil ist mit einem ersten Radbauteil gebildet, wobei die Rolleinrichtung betreibbar ist, dass das erste Radbauteil bei der Rollbewegung auf der Rollfläche des zweiten Rollbauteils und bei der weiteren Rollbewegung auf der weiteren Rollfläche des dritten Rollbauteils abrollt; und (ii) das erste Rollbauteil ist mit einem ersten und einem zweiten Radbauteil gebildet, die jeweils auf der Drehachse drehbar gelagert sind, wobei das erste und das zweite Radbauteil unterschiedliche Raddurchmesser aufweisen und die Rolleinrichtung betreibbar ist, dass das erste Radbauteil bei der Rollbewegung auf der Rollfläche des zweiten Rollbauteils und das zweite Radbauteil bei der weiteren Rollbewegung auf der weiteren Rollfläche des dritten Rollbauteils abrollt.

Ist das erste Rollbauteil mit einem ersten Radbauteil gebildet, kommt eine Lauffläche des ersten Radbauteils bei den Rollbewegungen sowohl mit der Rollfläche des zweiten Rollbauteils wie auch der weiteren Rollfläche des dritten Bauteils in Kontakt und rollt hierauf jeweils bei der Übertragung der Antriebsbewegung.

Das erste Rollbauteil kann mit dem ersten und dem zweiten Radbauteil als Doppelradbauteil ausgeführt sein. Das erste und das zweite Radbauteil können auf einer gemeinsamen Drehachse drehend gelagert sein. Es kann vorgesehen sein, dass das erste und das zweite Radbauteil drehfest miteinander verbunden sind, so dass eine Drehung des ersten Radbauteils das Mitdrehen des zweiten Radbauteils erzwingt, und umgekehrt. Das erste und das zweite Radbauteil rollen bei den Rollbewegungen auf den unterschiedlichen Rollflächen. Die Rollflächen, auf denen das erste und das zweite Radbauteil bei den Rollbewegungen rollen, können in Bezug auf Drehachse des ersten Rollbauteils auf gegenüberliegenden Seiten oder auf der gleichen Seite angeordnet sein.

Das erste Radbauteil kann einen kleineren Raddurchmesser als das zweite Radbauteil aufweisen. Hierdurch ist eine Ausführungsform bereitgestellt, einen mit Hilfe der Antriebsbewegung bereitgestellten Hub bei der Übertragung der Antriebsbewegung mittels der Übertragungseinrichtung zu ändern, also zu vergrößern oder zu verkleinern. Zum Beispiel kann der Hub der Antriebsbewegung mindestens verdoppelt werden.

Bei der Rolleinrichtung kann in einer Ausführungsform mindestens eine der folgenden Ausführungsformen vorgesehen sein: (i) die Rollfläche an dem zweiten Rollbauteil ist eine ebene Rollfläche; und (ii) die weitere Rollfläche an dem dritten Rollbauteil ist eine weitere ebene Rollfläche. Die ebene Rollfläche kann zum Beispiel an einer Stange oder einer Platte des jeweiligen Rollbauteils gebildet sein.

Bei der Rolleinrichtung kann in einer Ausführungsform mindestens eine der folgenden Ausführungsformen vorgesehen sein: (i) bei der Rollbewegung verbleibt eine Drehachse des ersten Rollbauteils in einer ortfesten Achsenlage im Modulgehäuse, während sich das zweite Rollbauteil mit der hieran gebildeten Rollfläche relativ zum Modulgehäuse verlagert, wobei das zweite Rollbauteil zum abtriebsseitigen Einkoppeln der übertragenen Antriebsbewegung mit dem Nadelschaft verbunden ist; und (ii) bei der Rollbewegung verbleibt das dritte Rollbauteil mit der hieran gebildeten weiteren Rollfläche in einer ortfesten Bauteillage im Modulgehäuse, während sich das erste Rollbauteil mit seiner Drehachse und das zweite Rollbauteil mit seiner Rollfläche relativ zum Modulgehäuse verlagern, wobei das erste oder das zweite Rollbauteil zum abtriebsseitigen Einkoppeln der übertragenen Antriebsbewegung mit dem Nadelschaft verbunden sind.

Bei der Ausführungsform, bei der die Drehachse des ersten Rollbauteils in der ortsfesten Achsenlage relativ zum Modulgehäuse verbleibt, wird die übertragene Antriebsbewegung abtriebsseitig an dem zweiten Rollbauteil bereitgestellt, um diese dann auf den Nadelschaft einzukoppeln, sei es direkt oder über ein oder mehrere Zwischenbauteile.

Verbleibt hingegen das dritte Rollbauteil relativ zum Modulgehäuse in der ortsfesten Bauteillage, wird die mittels der Rolleinrichtung übertragene Antriebsbewegung abtriebsseitig an dem ersten oder dem zweiten Rollbauteil abgreifbar bereitgestellt. Hierbei kann vorgesehen sein, dass die Antriebsbewegung antriebsseitig auf das erste Rollbauteil eingeleitet wird, so dass sich beispielweise die Drehachse des ersten Drehbauteils bei der Rollbewegung linear bewegt, insbesondere vor und zurück in Längsrichtung des Modulgehäuses.

Eine übertragene Linearbewegung kann abtriebsseitig abgegriffen werden, um sie auf den Nadelschaft einzukoppeln. In ähnlicher Weise bewirkt die Rollbewegung hierbei eine Linearbewegung des zweiten Rollbauteils, welche abtriebsseitig abgegriffen und auf den Nadelschaft einkoppelbar ist.

Die Linearbewegungen können jeweils parallel zur Längsrichtung des Modulgehäuses ausführbar sein.

Die Rolleinrichtung kann betreibbar sein, das dritte Rollbauteil mit der hieran gebildeten weiteren Rollfläche relativ zum Modulgehäuse zu verlagern, wenn bei der Rollbewegung die Drehachse des ersten Rollbauteils in der ortfesten Achsenlage im Modulgehäuse verbleiben, wobei das zweite und das dritte Rollbauteil hierbei in entgegengesetzte Bewegungsrichtungen verlagerbar sind.

Beim Verlagern des dritten Rollbauteils relativ zum Modulgehäuse bei ortsfester Achsenlage der Drehachse des ersten Rollbauteils kann das dritte Rollbauteil eine Linearbewegung in Längsrichtung des Modulgehäuses ausführen. Die Bewegung des dritten Rollbauteils relativ zum Modulgehäuse wird mit Hilfe der Rollbewegungen invertiert, so dass sich das zweite Rollbauteil in entgegengesetzter Bewegungsrichtung zur Bewegung des dritten Rollbauteils bewegt.

Auch bei dieser Ausführungsform kann das erste Rollbauteil mit dem ersten und dem zweiten Radbauteil gebildet sein, die unterschiedliche Raddurchmesser aufweisen. Bei dieser Ausführungsform mit dem ersten und dem zweiten Radbauteil können die sich in entgegengesetzter Richtung bewegenden Rollbauteile in Bezug auf die Drehachse des ersten Rollbauteils auf gegenüberliegenden Seiten oder auf der gleichen Seite angeordnet sein, wobei das erste Radbauteil auf der Rollfläche an dem zweiten Rollbauteil und das zweite Radbauteil auf der weiteren Rollfläche des dritten Rollbauteils rollt.

Die Rolleinrichtung kann betreibbar sein, das zweite und / oder das dritte Rollbauteil bei der Rollbewegung / der weiteren Rollbewegung zum Verlagern in dem Modulgehäuse linear zu bewegen. Die jeweilige lineare Bewegung kann in Längsrichtung des Modulgehäuses ausführbar sein.

Bei der Rolleinrichtung kann in einer Ausgestaltung eine der folgenden Ausführungsformen vorgesehen sein: (i) mit dem ersten Rollbauteil ist ein antriebsseitiges Rollbauteil gebildet, auf welches die Antriebsbewegung einkoppelbar ist, und mit dem zweiten oder dem dritten Rollbauteil ist ein abtriebsseitiges Rollbauteil gebildet, welches betreibbar ist, die übertragene Antriebsbewegung abtriebsseitig auf den Nadelschaft einzukoppeln; und (ii) mit dem zweiten oder dem dritten Rollbauteil ist ein antriebsseitiges Rollbauteil gebildet, auf welches die Antriebsbewegung einkoppelbar ist, und mit dem ersten Rollbauteil ist ein abtriebsseitiges Rollbauteil gebildet, welches betreibbar ist, die übertragene Antriebsbewegung abtriebsseitig auf den Nadelschaft einzukoppeln.

Ist mit dem ersten Rollbauteil das antriebsseitige Rollbauteil gebildet, so erfolgt das Einkoppeln der Antriebsbewegung mit der Wiederholfrequenz auf das erste Rollbauteil. Abtriebsseitig ist die übertragene Antriebsbewegung an dem zweiten oder dem dritten Rollbauteil abgreifbar und sodann auf dem Nadelschaft einkoppelbar.

Bilden das zweite oder das dritte Rollbauteil das antriebsseitige Rollbauteil, erfolgt das Einkoppeln der Antriebsbewegung mit der Wiederholfrequenz antriebsseitig auf das zweite oder das dritte Rollbauteil. Abtriebsseitig ist die übertragende Antriebsbewegung an dem ersten Rollbauteil abgreifbar, zum Beispiel in Form der linearen Hin- und Herbewegung der Drehachse des ersten Rollbauteils, insbesondere in Längsrichtung des Modulgehäuses.

Das abtriebsseitige Bauteil, an dem die übertragende Antriebsbewegung abgreifbar ist, kann mit dem Nadelschaft einstückig ausgeführt sein, zum Beispiel als Spritzgussbauteil.

Bei der Rolleinrichtung kann mindestens eine der folgenden Ausführungsformen vorgesehen sein: (i) zwischen dem ersten und dem zweiten Rollbauteil ist eine Reibschlussverbindung ausgebildet, und (ii) zwischen dem ersten und dem dritten Rollbauteil ist eine Reibschlussverbindung ausgebildet. Mit Hilfe der Reibschlussverbindung wird ein Kraftschluss zwischen den an der jeweiligen Rollbewegung beteiligten Rollbauteilen ausgebildet. Zum Ausbilden der Reibschlussverbindung können an den Rollbauteilen rutschhemmenden Oberflächen vorgesehen sein, zum Beispiel Gummioberflächen oder aufgeraute Oberflächen. Die bei der Rollbewegung in Kontakt tretenden Oberflächen der Rollbauteile können im Wesentlichen glatte Oberflächen sein, insbesondere zahnfreie Oberflächen.

Bei der Rolleinrichtung kann bei einer Ausführungsform mindestens eine der folgenden Ausführungsformen vorgesehen sein: (i) zwischen dem ersten und dem zweiten Rollbauteil ist eine Formschlussverbindung ausgebildet, und (ii) zwischen dem ersten und dem dritten Rollbauteil ist eine Formschlussverbindung ausgebildet.

Zum Ausbilden der Formschlussverbindung können die beteiligten Rollbauteile einander zugeordnete Verzahnungen im Bereich der Oberflächen aufweisen, die bei der jeweiligen Rollbewegung in Kontakt kommen. Bei dem ersten Rollbauteil kann es sich zum Beispiel um ein Einfach- oder Doppelzahnrad handeln. Das zweite und / oder das dritte Rollbauteil können in einer Ausführungsform mit Hilfe einer Zahnstange gebildet sein. In Verbindung mit der Verzahnung kann eine Schrägverzahnung oder eine Doppel-Schrägverzahnung vorgesehen sein. Eine Schrägverzahnung sieht insbesondere eine nicht zur Rotationsachse parallele Anordnung der Zähne beim Zahnrad vor.

Die Rolleinrichtung kann betreibbar sein, die Rollbewegung und / oder die weitere Rollbewegung als reines Rollen auszuführen. Das reine Rollen sorgt zum Beispiel für ein schlupffreies Rollen bei der jeweiligen Rollbewegung.

Die Übertragungseinrichtung kann eingerichtet sein, eine lineare repetierende Antriebsbewegung mit der Wiederholfrequenz zu übertragen. Mit Hilfe der linearen repetierenden Antriebsbewegung kann ein antriebsseitiger Hub (Antriebshub) bereitgestellt werden. In Bezug auf die Übertragungseinrichtung kann abtriebsseitig ein Stechhub für das Aus- und Einfahren der wenigstens einen Stechnadel bereitgestellt werden, wobei Antriebshub und Stechhub hinsichtlich der jeweiligen Hublänge und / oder des jeweiligen Bewegungsrichtungssinnes gleich oder verschieden sein können.

Mittels der unterschiedlichen Ausführungen der Übertragungseinrichtung kann die antriebsseitig bereitgestellte Antriebsbewegung (Antriebshub) invertiert und / oder skaliert übertragen und auf die Stecheinrichtung eingekoppelt werden (Stechhub).

In einer Ausführung kann das dritte Rollbauteil auf gegenüberliegenden Seiten des ersten Rollbauteils mit der Drehachse ausgebildet sein und auf den gegenüberliegenden Seiten jeweils einen Abschnitt der weiteren Rollfläche aufweisen, auf der das erste Roll bauteil beim Übertragen der Antriebsbewegung rollt. Auch das zweite Rollbauteil kann auf gegenüberliegenden Seiten des ersten Rollbauteils mit der Drehachse ausgebildet sein und auf den gegenüberliegenden Seiten jeweils einen Abschnitt der Rollfläche aufweisen, auf der das erste Rollbauteil rollt. Bei einem solchen Beispiel sind somit das zweite und das dritte Rollbauteil jeweils auf gegenüberliegenden Seiten des ersten Rollbauteils gebildet, wobei das erste Rollbauteil beim Übertragen der Antriebsbewegung auf den gegenüberliegenden Seiten auf getrennten Abschnitten der Rollfläche sowie getrennten Abschnitten der weiten Rollfläche rollt.

Das erste Rollbauteil kann in einem Ausführungsbeispiel aus zwei voneinander unabhängigen Radbauteilen gleicher Geometrie gebildet sein, die auf derselben Drehachse oder unterschiedlichen Drehachsen angeordnet sind. Beide Radbauteile des ersten Rollbauteils können bei einer Ausführung auf in Bewegungsrichtung parallel versetzten Drehachsen angeordnet sein.

Das Nadelmodul kann in einer Ausführungsform eine Rückstelleinrichtung aufweisen, die eingerichtet oder betreibbar ist, eine Rückstellkraft für den Nadelschaft bereitzustellen, um eine Rückbewegung der wenigstens einen Stechnadel aus der ausgefahrenen Stellung zu unterstützen oder allein zu bewirken. Hierbei kann vorgesehen sein, dass die Rückstelleinrichtung gegen das Ausfahren der wenigstens einen Stechnadel in die ausgefahrene Stellung vorgespannt ist, so dass die Vorspannkraft die Rückbewegung der wenigstens einen Stechnadel aus der ausgefahrenen Stellung bewirkt oder zumindest unterstützt. In einer Ausführungsform weist die Rückstelleinrichtung eine Zug- oder eine Druckfeder auf, welche den Nadelschaft oder die wenigstens eine Stechnadel gegen das Bewegen in die ausgefahrene Stellung vorspannt.

In einer Ausgestaltung kann die Rückstelleinrichtung mittels der Dichtmembran, welche die rückseitige Modulöffnung dichtend verschließt, gebildet sein, derart, dass die Dichtmembran elastisch gestreckt oder gedehnt wird, wenn die wenigstens eine Stechnadel in die ausgefahrene Stellung verlagert wird, so dass die elastische Membrankraft (Vorspann- oder Rückstellkraft) dann die wenigstens eine Stechnadel aus der ausgefahrenen Stellung zurückzieht.

Bei einem Ausführungsbeispiel kann das dritte Rollbauteil auf gegenüberliegenden Seiten des ersten Rollbauteils mit der Drehachse ausgebildet sein und auf den gegenüberliegenden Seiten jeweils einen Abschnitt der weiteren Rollfläche aufweisen, auf der das erste Rollbauteil jeweils rollt. Auch das zweite Rollbauteil kann auf gegenüberliegenden Seiten des ersten Rollbauteils mit der Drehachse ausgebildet sein und auf den gegenüberliegenden Seiten jeweils einen Abschnitt der Rollfläche aufweisen, auf der das erste Rollbauteil rollt. Somit können das zweite und das dritte Rollbauteil jeweils auf gegenüberliegenden Seiten des ersten Rollbauteils gebildet sein, wobei das erste Rollbauteil auf den gegenüberliegenden Seiten auf getrennten Abschnitten der Rollfläche sowie der weiteren Rollfläche rollt. Hierbei kann das erste Rollbauteil zweistückig mit einem oberen ersten Rollbauteil, insbesondere einem oberen ersten Radbauteil, und einem unteren ersten Rollbauteil, insbesondere einem unteren ersten Radbauteil, gebildet sein, die auf der gemeinsamen Drehachse drehbar gelagert und oberhalb sowie unterhalb einer Trenn-Gleitfläche angeordnet sind.

Bei den unterschiedlichen Ausführungen zum Übertragen der Antriebsbewegung können die Funktionen von Antrieb (insbesondere Empfangen der Antriebsbewegung) und Abtrieb konstruktiv getauscht sein, um ein für die jeweils gewünschte Anwendung geeignetes Übertragungsverhältnis auszubilden.

Die in Verbindung mit dem Nadelmodul erläuterten Ausgestaltungen können im Zusammenhang mit dem Handgerät zum lokalen Aufstechen einer menschlichen oder einer tierischen Haut entsprechend vorgesehen sein.

Auch können einzelne oder mehrere der vorangehend für das Nadelmodul beschriebenen Ausgestaltungen bei dem Verfahren zum Betreiben des Nadelmoduls entsprechende Anwendung finden.

### Beschreibung von Ausführungsbeispielen

Im Folgenden werden weitere Ausführungsbeispiele unter Bezugnahme auf Figuren einer Zeichnung erläutert. Hierbei zeigen:
- Fig. 1: eine schematische perspektivische Darstellung eines Handgeräts zum lokalen Aufstechen einer menschlichen oder einer tierischen Haut;
- Fig. 2: eine schematische perspektivische Darstellung des Handgeräts aus Fig. 1 teilweise im Aufriss, wobei ein Nadelmodul von einem Handstück des Handgeräts getrennt gezeigt ist;
- Fig. 3: eine schematische perspektivische Darstellung eines weiteren Nadelmoduls für ein Handgerät zum lokalen Aufstechen einer menschlichen oder einer tierischen Haut mit Stechnadeln in einer eingefahrenen Stellung;
- Fig. 4: eine schematische perspektivische Darstellung des weiteren Nadelmoduls aus Fig. 3 mit den Stechnadeln in einer ausgefahrenen Stellung;
- Fig. 5: eine schematische perspektivische Darstellung eines Nadelmoduls vergleichbar der Ausführung in den Fig. 1 und 2 mit Stechnadeln in einer eingefahrenen Stellung im Schnitt;
- Fig. 6: eine schematische perspektivische Darstellung des Nadelmoduls aus Fig. 5 mit den Stechnadeln in der ausgefahrenen Stellung im Schnitt;
- Fig. 7: eine schematische Darstellung einer Anordnung für eine Rolleinrichtung einer Übertragungseinrichtung zum Übertragen einer mit einer Wiederholfrequenz bereitgestellten Antriebsbewegung;
- Fig. 8: eine schematische Darstellung einer Anordnung für eine weitere Rolleinrichtung einer Übertragungseinrichtung zum Übertragen einer mit einer Wiederholfrequenz bereitgestellten Antriebsbewegung mit einem Doppelrad;
- Fig. 9: eine schematische Darstellung einer Anordnung für eine alternative Rolleinrichtung einer Übertragungseinrichtung zum Übertragen einer mit einer Wiederholfrequenz bereitgestellten Antriebsbewegung mit einem Doppelrad;
- Fig. 10: eine schematische Darstellung einer Anordnung für eine andere Rolleinrichtung einer Übertragungseinrichtung zum Übertragen einer mit einer Wiederholfrequenz bereitgestellten Antriebsbewegung mit Doppelrad;
- Fig. 11: eine schematische Darstellung einer Anordnung für eine noch andere Rolleinrichtung einer Übertragungseinrichtung zum Übertragen einer mit einer Wiederholfrequenz bereitgestellten Antriebsbewegung mit Doppelrad;
- Fig. 12: eine schematische perspektivische Darstellung des weiteren Nadelmoduls in der Ausführung gemäß Fig. 3 mit Stechnadeln in einer eingefahrenen Stellung;
- Fig. 13: eine schematische perspektivische Darstellung des weiteren Nadelmoduls aus Fig. 12, wobei Teile eines Modulgehäuses weggelassen sind;
- Fig. 14: eine schematische perspektivische Darstellung des weiteren Nadelmoduls aus Fig. 13 weiter im Detail;
- Fig. 15: eine schematische perspektivische Darstellung des weiteren Nadelmoduls aus Fig. 12 mit Stechnadeln in der ausgefahrenen Stellung;
- Fig. 16: eine schematische perspektivische Darstellung des weiteren Nadelmoduls aus Fig. 15, wobei das Modulgehäuse teilweise weggelassen ist; und
- Fig. 17: eine schematische perspektivische Darstellung des weiteren Nadelmoduls aus Fig. 16 weiter im Detail.

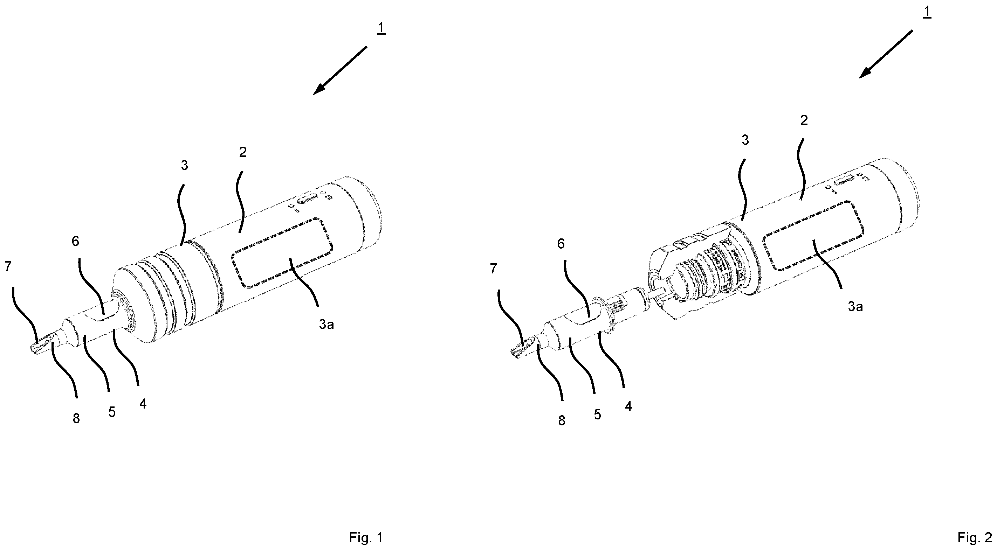

Fig. 1 und 2 zeigen schematische perspektivische Darstellungen eines Handgeräts 1 zum lokalen Aufstechen einer menschlichen oder einer tierischen Haut. Das Handgerät 1 weist ein Handstück 2 auf, welches im Betrieb von einem Nutzer mit den Fingern gegriffen werden kann. In einem Modulgehäuse 3 des Handstücks 2 ist eine Antriebseinrichtung 3a angeordnet, insbesondere ein elektrischer Motor, mit der eine Antriebsbewegung mit einer Wiederholfrequenz (repetierend) bereitgestellt wird. Zum Beispiel kann der elektrische Motor eine Drehbewegung bereitstellen, die mit Hilfe eines Wandlungsmechanismus (nicht dargestellt) in eine lineare Vor- und Zurückbewegung gewandelt wird, wie dies für verschiedene Ausführungsformen als solches bekannt ist.

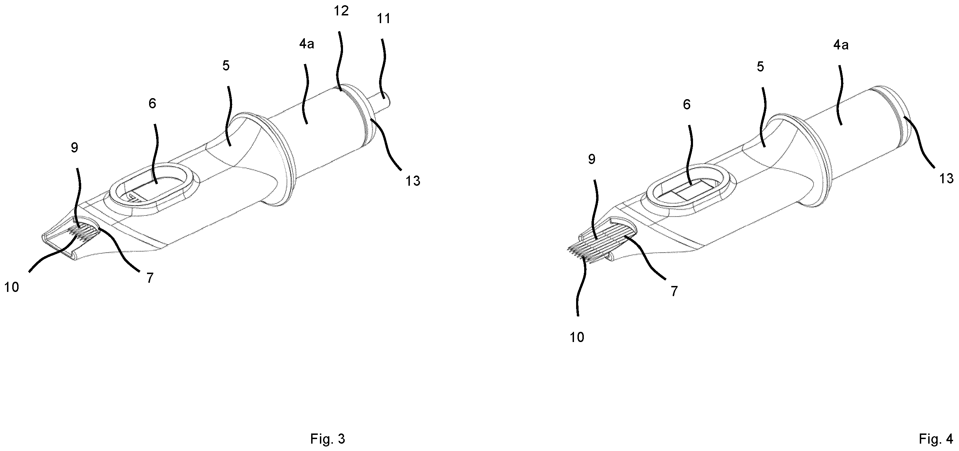

An dem Handstück 2 ist frontseitig ein Nadelmodul 4 lösbar angeordnet, welches mit einem Modulgehäuse 5 gebildet ist. Das Modulgehäuse 5, welches zum Beispiel als Spritzgussbauteil ausgeführt ist, weist eine Einfüllöffnung 6 auf, über die im Betrieb ein Stoff eingeführt werden kann, derart, dass der eingeführte Stoff beim lokalen Aufstechen der Haut in diese eingebracht wird, zum Beispiel ein Farbstoff, ein medizinischer oder ein kosmetischer Wirkstoff. Hierzu werden im Betrieb durch eine frontseitige Modulöffnung 7 an einer Modulspitze 8 Stechnadeln 9 (vgl. Fig. 3 und 4) aus- und eingefahren.

Gemäß Fig. 2 ist das Nadelmodul 4 lösbar an dem Handstück 2 angeordnet, so dass das Nadelmodul 4 zum Beispiel als Einwegmodul ausbildbar ist, welches nach einer Stechbehandlung abgenommen und entsorgt werden kann.

Fig. 3 und 4 zeigen schematische perspektivische Darstellungen eines weiteren Nadelmoduls 4a, wobei die Stechnadeln 9 in Fig. 3 in einer eingefahrenen Stellung und in Fig. 4 in einer ausgefahrenen Stellung gezeigt sind, in welcher Stechspitzen 10 außerhalb des Modulgehäuses 5 und gegenüber der frontseitigen Modulöffnung 7 vorstehend angeordnet sind.

Zum Aus- und Einfahren der Stechnadeln 9 wird von der Antriebseinrichtung 3a im Handstück 2 repetierend eine Antriebskraft oder -bewegung bereitgestellt, welche über ein Kopplungsbauteil 11 (vgl. Fig. 3) in das weitere Nadelmodul 4a übertragen oder eingekoppelt wird. Das Kopplungsbauteil 11 erstreckt sich bei der gezeigten Ausführungsform durch eine rückseitige Modulöffnung 12, die mittels einer Dichtmembran 13 verschlossen und abgedichtet ist. Das Kopplungsbauteil 11 ist durch die Dichtmembran 13 gedichtet hindurchgeführt. Die Dichtmembran 13 ist aus einem elastisch dehnbaren Material, zum Beispiel einem Gummi- oder Kunststoffmaterial. Wird das Kopplungsbauteil 11 aufgrund der Einleitung der Antriebsbewegung in der rückseitigen Modulöffnung 12 nach vorn in Richtung der frontseitigen Modulöffnung 7 bewegt, erfolgt hierdurch eine Streckung von Abschnitten der Dichtmembran 13 in Richtung der frontseitigen Modulöffnung 7. Aufgrund dieser Streckung oder Dehnung wird eine Rückstellkraft bereitgestellt, mit der das Kopplungsbauteil 11 nach der Vorwärtsbewegung zurückgezogen wird, was eine Zurückbewegung der Stechnadeln 9 aus der ausgefahrenen Stellung in Fig. 4 in die eingefahrene Stellung gemäß Fig. 3 bewirkt oder zumindest unterstützt. Auf diese Weise ist mit Hilfe der Dichtmembran 13 eine Rückstelleinrichtung gebildet, mit der eine Vorspannkraft gegen die Vorwärtsbewegung des Kopplungsbauteils 11 bereitgestellt ist.

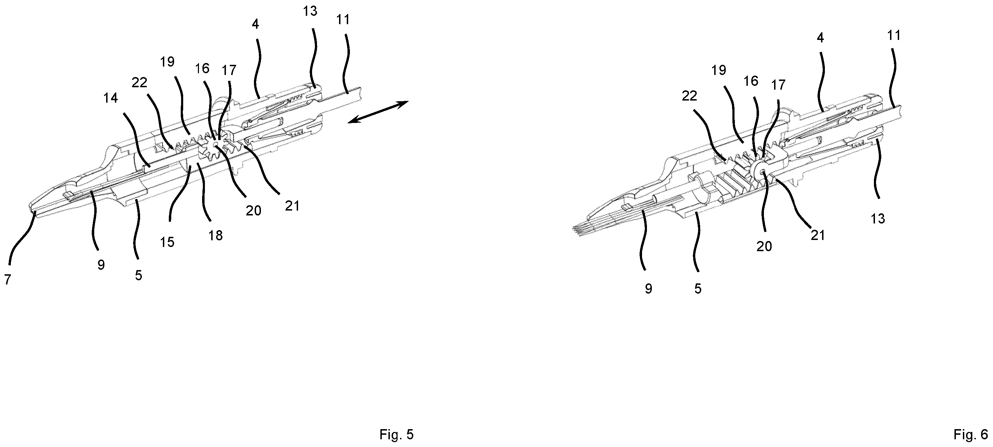

Fig. 5 und 6 zeigen schematische perspektivische Darstellungen des Nadelmoduls 4, vergleichbar der Ausführung in den Fig. 1 und 2, mit Stechnadeln 9 in einer eingefahrenen Stellung und einer ausgefahrenen Stellung, jeweils im Schnitt. Für gleiche Merkmale werden in den Fig. 5 und 6 dieselben Bezugszeichen wie in den vorangehenden Fig. 1 bis 4 verwendet.

Es ergibt sich, dass die Stechnadeln 9 an einem Nadelschaft 14 angeordnet sind, welcher im Betrieb aufgrund der mit der Wiederholfrequenz bereitgestellten Antriebsbewegung in Längsrichtung des Modulgehäuses 5 vor- und zurückbewegt wird, so dass die Stechnadeln 9 wiederholt zwischen der ausgefahrenen und der eingefahrenen Stellung verlagert werden. Das Modulgehäuse 5 weist bei dem Nadelmodul 4 in den Fig. 5 und 6 eine im Wesentlichen runde Außenform auf, wohingegen es bei der Ausführung in den Fig. 3 und 4 mit einer Flachform gebildet ist.

Zum Übertragen der über das Kopplungsbauteil 11 eingekoppelten linearen Antriebsbewegung auf den Nadelschaft 14 ist eine Übertragungseinrichtung 15 vorgesehen. Die Übertragungseinrichtung 15 weist eine Rolleinrichtung 16 auf, die im gezeigten Beispiel mit einem ersten, einem zweiten und einem dritten Rollbauteil 17, 18, 19 gebildet ist. Das erste Rollbauteil 17 ist auf einer Drehachse 20 drehbar gelagert. Das zweite Rollbauteil 18, welches im dargestellten Beispiel mit einer Zahnstange gebildet ist, ist in dem Modulgehäuse 5 in Längsrichtung des Modulgehäuses 5 verlagerbar aufgenommen. An dem zweiten Rollbauteil 18 ist eine Rollfläche 21 dem ersten Rollbauteil 17, welches mit einem Zahnrad ausgeführt ist, gegenüberliegend gebildet.

Das dritte Rollbauteil 19, welches bei dem gezeigten Ausführungsbeispiel ebenfalls mit einer Zahnstange gebildet ist, ist in dem Modulgehäuse 5 ortsfest gelagert und weist eine weitere Rollfläche 22 auf, die dem ersten Rollbauteil 17 zugewandt ist.

Das erste Rollbauteil 17 ist mit dem Kopplungsbauteil 11 verbunden, derart, dass die Antriebsbewegung (Antriebshub) auf das erste Rollbauteil 17 eingekoppelt werden kann, so dass sich dieses mit seiner Drehachse 20 in Längsrichtung des Modulgehäuses 5 verlagert (Linearbewegung), wobei sich das erste Rollbauteil 17 hierbei auf der Drehachse 20 dreht, so dass das zweite Rollbauteil 18 in Längsrichtung des Modulgehäuses 5 verlagert wird, wie dies die Fig. 5 und 6 für die eingefahrene und die ausgefahrene Stellung der Stechnadeln 9 zeigen. Zur Übertragung der Antriebsbewegung rollt das erste Rollbauteil 17 auf der Rollfläche 21 an dem zweiten Rollbauteil 18, wie auch auf der weiteren Rollfläche 22 an dem dritten (ortsfesten) Rollbauteil 19, so dass jeweilige Rollbewegungen ausgeführt werden. Bei dem Ausführungsbeispiel in den Fig. 5 und 6 ist zwischen den Rollbauteilen mit Hilfe des Zahnrads und den Zahnstangen eine Formschlussverbindung ausgebildet.

Gemäß Fig. 6 ist das zweite Rollbauteil 18 aufgrund der Rollbewegung infolge der eingekoppelten Antriebsbewegung in dem Modulgehäuse 5 nach vorn bewegt, wie auch der hiermit verbundene Nadelschaft 14 sowie die am Nadelschaft 14 angeordneten Stechnadeln 9.

Bewegt sich das Kopplungsbauteil 11 mit den hiermit verbundenen ersten Rollbauteil 17 wieder zurück, rollt das erste Rollbauteil 17 auf der weiteren Rollfläche 22 des dritten Rollbauteils 19 zurück, wodurch aufgrund der Rollbewegung des ersten Rollbauteils 17 auf der Rollfläche 21 des zweiten Rollbauteils 18 auch dieses im Modulgehäuse 5 zurückbewegt wird, so dass schließlich die Stechnadeln 9 in der eingefahrenen Stellung gemäß Fig. 5 angeordnet sind.

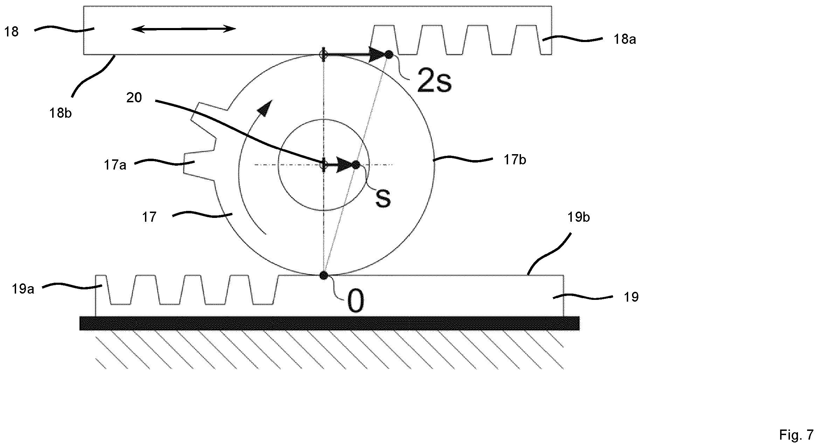

Fig. 7 zeigt eine schematische Darstellung einer Anordnung für die Rolleinrichtung 16 mit dem ersten, dem zweiten und dem dritten Rollbauteil 17,18, 19. Das dritte Rollbauteil 19 ist ortsfest im Modulgehäuse 5 angeordnet, so dass eine Einleitung des Antriebshub (repetierende Antriebsbewegung) auf das erste Rollbauteil 17 und eine hierdurch bewirkte lineare Bewegung der Drehachse 20 um den Weg s eine lineare Bewegung von 2s des zweiten Rollbauteils 18 verursacht.

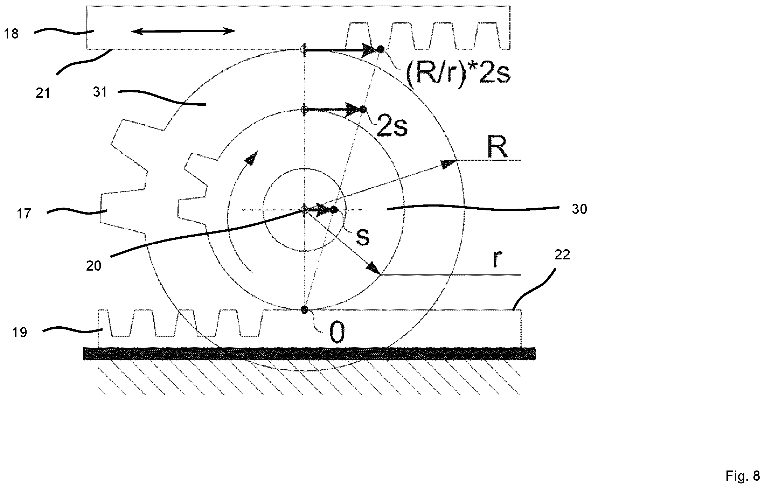

In unterschiedlichen Ausführungen können das erste Rollbauteil 17 sowie das zweite und das dritte Rollbauteil 18, 19 jeweils eine Verzahnung 17a, 18a, 19a (Formschlussverbindung der Rollbauteile) aufweisen, was in Fig. 7 schematisch angedeutet ist (ähnlich in den Fig. 8 bis 10). Alternativ weisen die Rollbauteile einander für die Rollbewegung zugeordnete zahnfreie oder glatte Oberflächen 17b, 18b, 19b (Reibschlussverbindung der Rollbauteile) auf, was in Fig. 7 ebenfalls schematisch angedeutet ist. Jeweils handelt es sich bei den Rollbewegungen auf den Rollflächen 21, 22 zum Beispiel um reines Rollen. Die vorangehenden Erläuterungen gelten entsprechend für weitere Ausführungsbeispiele in den folgenden Fig. 8 bis 10.

Fig. 8 zeigt eine schematische Darstellung für eine andere Anordnung der Rolleinrichtung 16, bei der das erste Rollenbauteil 17 mit einem ersten Radbauteil 30 und einem zweiten Radbauteil 31 gebildet ist, die unterschiedliche Radien r und R aufweisen, also unterschiedliche Raddurchmesser. Während das erste Radbauteil 30 beim Einleiten der Antriebsbewegung auf das erste Rollbauteil 17 auf der weiteren Rollfläche 22 des dritten Rollbauteils 19 rollt, findet die Rollbewegung auf der Rollfläche 21 des zweiten Rollbauteils 18 durch das zweite Radbauteil 31 statt. Auf diese Weise kann der Antriebshub s, also die auf die Drehachse 20 eingeleitete lineare Antriebsbewegung vergrößert werden zu (R / r) x 2s.

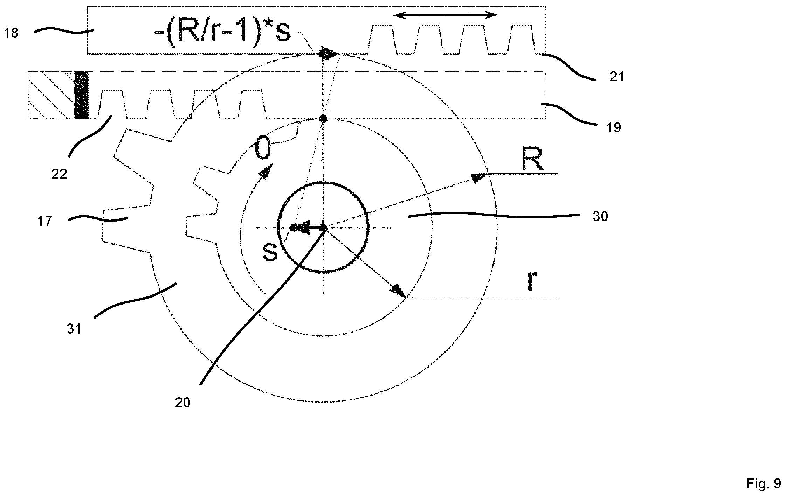

Fig. 9 zeigt eine schematische Darstellung für eine alternative Anordnung der Rolleinrichtung 16, bei der das erste Rollenbauteil 17 mit dem ersten Radbauteil 30 und dem zweiten Radbauteil 31 gebildet ist, die unterschiedliche Radien r und R aufweisen, also unterschiedliche Raddurchmesser. Das zweite Rollbauteil 18 und das dritte Rollbauteil 19 sind in Bezug auf die Drehachse 20 des ersten Rollbauteils auf der gleichen Seite angeordnet, im gezeigten Beispiel oberhalb. Das dritte Rollbauteil 19 ist ortsfest gelagert. Infolge des Einleitens der Antriebsbewegung auf das erste Rollbauteil 17 rollt das erste Radbauteil 30 auf der weiteren Rollfläche 22 des dritten Rollbauteils 19. Die Rollbewegung auf der Rollfläche 21 des zweiten Rollbauteils 18 führt das zweite Radbauteil 31 aus. Auf diese Weise kann der Antriebshub s, also die auf die Drehachse 20 eingeleitete lineare Antriebsbewegung verändert oder übersetzt werden zu -(R / r-1) x s.

Alternativ kann die Antriebsbewegung s auf das zweite Rollbauteil 18 eingeleitet und auf die Verlagerung der Drehachse 20 des ersten Rollbauteils 17 mit -r × s / (R - r) übertragen werden (nicht dargestellt).

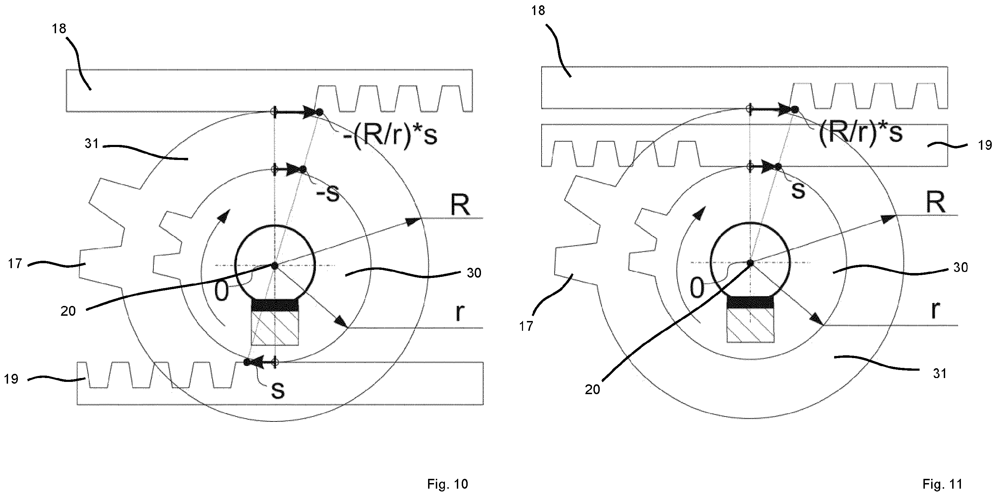

Die Fig. 10 zeigt eine weitere Anordnung für eine alternative Ausgestaltung der Rolleinrichtung 16, bei der das erste Rollbauteil 17 in dem Modulgehäuse 5 des Nadelmoduls ortsfest gelagert ist, so dass sich bei der Rollbewegung zur Übertragung der Antriebsbewegung das zweite und das dritte Rollbauteil 18, 19, die beide axial bewegbar sind, in gleicher Richtung mit entgegengesetztem Richtungssinn bewegen (Inverteranordnung). Das zweite und das dritte Rollbauteil 18, 19 sind gemäß dem Ausführungsbeispiel in Fig. 10 in Bezug auf die Drehachse 20 des ersten Rollbauteils 17 auf gegenüberliegenden Seiten angeordnet sein.

Die Beispiele zeigen, dass mittels unterschiedlicher Ausführungen der Übertragungseinrichtung 15 die antriebsseitig bereitgestellte Antriebsbewegung (Antriebshub) invertiert und / oder skaliert übertragen und abtriebsseitig auf den Nadelschaft 14 (Stechhub) eingekoppelt werden kann.

Bei den unterschiedlichen Ausführungen zum Übertragen der Antriebsbewegung können die Funktionen von Antrieb (Empfangen der Antriebsbewegung) und Abtrieb konstruktiv getauscht sein, um ein für die jeweils gewünschte Anwendung geeignetes Übertragungsverhältnis auszubilden.

Eine andere Anordnung mit ortsfester Lage der Drehachse 20 des ersten Rollbauteils 17 zeigt Fig. 11. Hier sind das zweite und das dritte Rollbauteil 18, 19, die jeweils verlagerbar im Modulgehäuse 5 angeordnet sind, auf der gleichen Seite in Bezug auf die Drehachse 20 angeordnet.

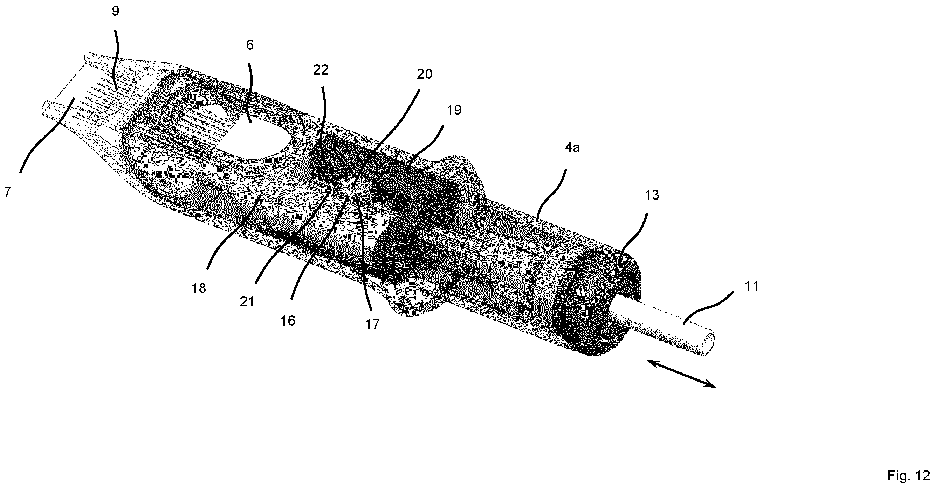

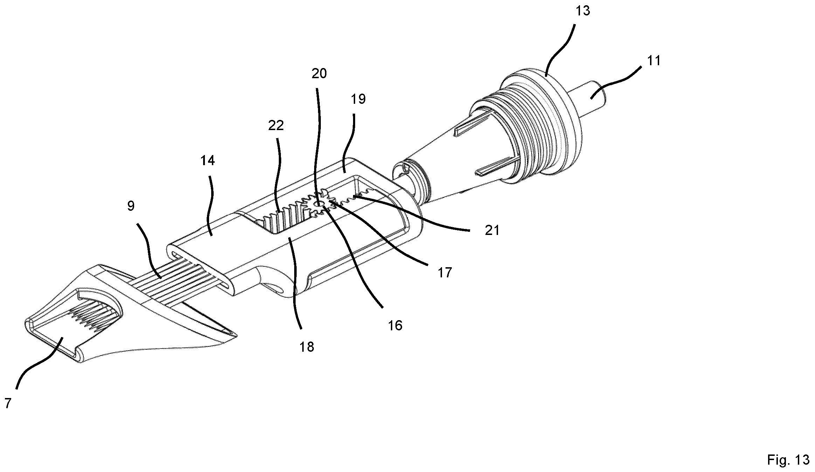

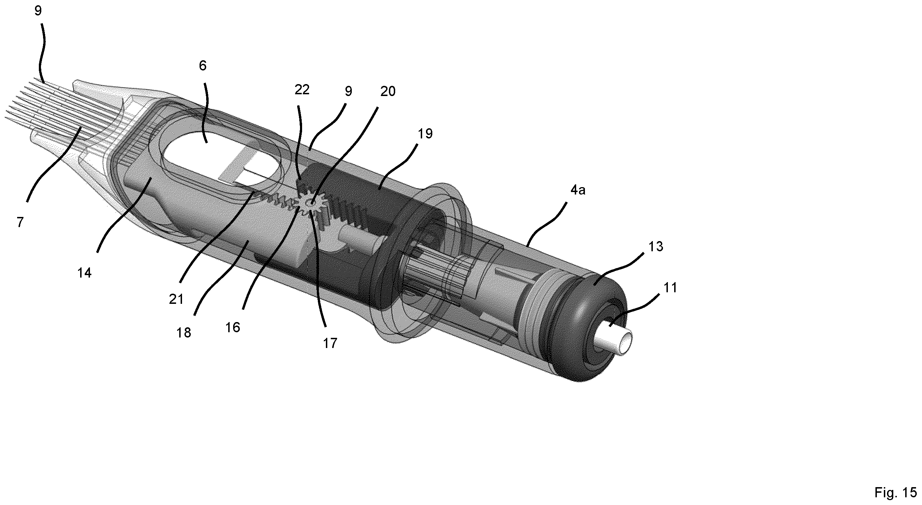

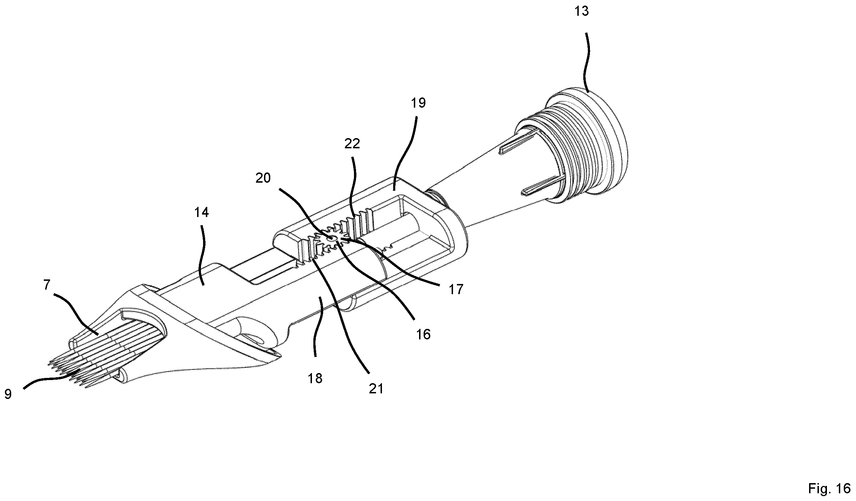

Die Fig. 12 bis 17 zeigen schematische perspektivische Darstellungen einer Anordnung für das weitere Nadelmodul 4a in der Ausführung nach den Fig. 3 und 4 mit dem Stechnadeln 9 in der eingefahrenen Stellung (vgl. Fig. 12 bis 14) sowie der ausgefahrenen Stellung (vgl. Fig. 15 bis 17). Für gleiche Merkmale werden in den Fig. 12 bis 17 dieselben Bezugszeichen wie in den Fig. 1 bis 6 verwendet.

Der Nadelschaft 14, an dem die Stechnadeln 9 angeordnet sind, ist mit dem zweiten Rollbauteil 18 einstückig verbunden. Beim antriebsseitigen Einkoppeln der Antriebsbewegung, welche mittels des Kopplungsbauteils übertragen wird, bewegt sich das erste Rollbauteil 17 mit der Drehachse 20 in Längsrichtung des Modulgehäuses 5 und rollt hierbei auf der Rollfläche 21 sowie der weiteren Rollfläche 22. Aufgrund der ortsfesten Lage des dritten Rollbauteils 19 bewegt sich das zweite Rollbauteil 18 linear vor und zurück. Die Bewegung wird abtriebsseitig auf den Nadelschaft 14 eingekoppelt, welcher sich gemeinsam mit dem zweiten Rollbauteil 18 bewegt.

Wird das Kopplungsbauteil 11 aufgrund der eingekoppelten Antriebsbewegung nach vorn in Richtung der frontseitigen Modulöffnung 7 bewegt, werden Membranabschnitte 13a der Dichtmembran 13, die das Kopplungsbauteil 11 abschnittsweise umgreifen und sich ihrerseits im Wesentlichen in Längsrichtung des Modulgehäuses erstrecken, gedehnt, wodurch eine Rückstellkraft für das Zurückbewegung des Kopplungsbauteils 11 und somit eine Rückbewegung des ersten Rollbauteils 17 entsteht.

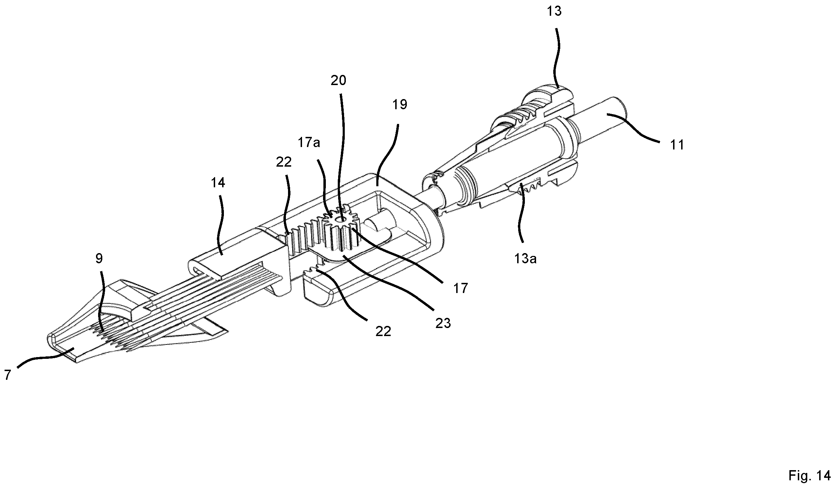

Gemäß Fig. 14 ist das dritte Rollbauteil 19 auf gegenüberliegenden Seiten des ersten Rollbauteils 17 mit der Drehachse 20 ausgebildet und weist auf den gegenüberliegenden Seiten jeweils einen Abschnitt der weiteren Rollfläche 22 auf, auf der das erste Rollbauteil 17 rollt.

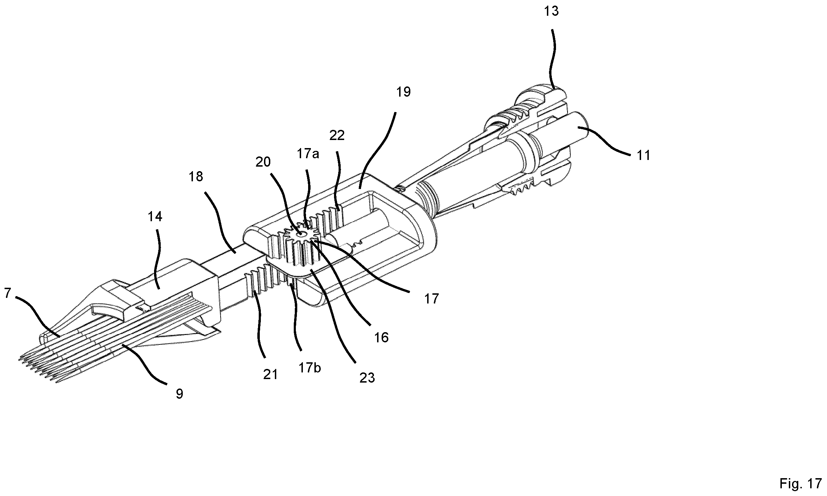

Gemäß Fig. 17 ist auch das zweite Rollbauteil 18 auf gegenüberliegenden Seiten des ersten Rollbauteils 17 mit der Drehachse 20 ausgebildet und weist auf den gegenüberliegenden Seiten jeweils einen Abschnitt der Rollfläche 21 auf, auf der das erste Rollbauteil 17 rollt. Beim gezeigten Beispiel sind somit das zweite und das dritte Rollbauteil 18, 19 jeweils auf gegenüberliegenden Seiten des ersten Rollbauteils 17 gebildet, wobei das erste Rollbauteil 17 auf den gegenüberliegenden Seiten auf getrennten Abschnitten der Rollfläche 21 sowie der weiteren Rollfläche 22 rollt. Hierbei ist das erste Rollbauteil 17 zweistückig mit einem oberen ersten Radbauteil 17a und einem unteren ersten Radbauteil 17b gebildet, die auf der gemeinsamen Drehachse 20 drehbar gelagert und oberhalb sowie unterhalb einer Trenn-Gleitfläche 23 angeordnet sind.

Die in der vorstehenden Beschreibung, den Ansprüchen sowie der Zeichnung offenbarten Merkmale können sowohl einzeln als auch in beliebiger Kombination für die Verwirklichung der verschiedenen Ausführungen von Bedeutung sein.

## Patentansprüche

1. Nadelmodul (4; 4a) für ein Handgerät zum lokalen Aufstechen einer menschlichen oder einer tierischen Haut, mit:
- einem Modulgehäuse (5), welches eine Modulspitze und eine im Bereich der Modulspitze ausgebildete frontseitige Modulöffnung (7) aufweist;
- einer Stecheinrichtung, welche wenigstens eine an einem Nadelschaft (14) angeordnete Stechnadel (9) mit einer Nadelspitze (10) aufweist und in dem Modulgehäuse (5) aufgenommen ist, derart, dass die wenigstens eine Stechnadel (9) mittels einer linearen Bewegung zwischen einer eingefahrenen und einer ausgefahrenen Stellung verlagerbar ist, wobei sich die wenigstens eine Stechnadel (9) zumindest in der ausgefahrenen Stellung durch die fronseitige Modulöffnung (7) erstreckt und die Nadelspitze hierbei außerhalb des Modulgehäuses (5) angeordnet ist; und
- einer Übertragungseinrichtung, die
- wenigstens teilweise in dem Modulgehäuse (5) aufgenommen und betreibbar ist, eine antriebsseitig mit einer Wiederholfrequenz bereitgestellte Antriebsbewegung auf den Nadelschaft (14) zu übertragen, derart, dass die übertragene Antriebsbewegung abtriebsseitig auf die wenigstens eine Stechnadel (9) einkoppelbar ist, um diese wiederholt zwischen der eingefahrenen und der ausgefahrenen Stellung zu verlagern; und
- eine Rolleinrichtung (16) aufweist, die ein erstes Rollbauteil (17), welches drehbar gelagert ist, und ein zweites Rollbauteil (18) aufweist und auf die Antriebsbewegung einleitbar ist, derart, dass beim Übertragen der Antriebsbewegung eine Rollbewegung ausgeführt wird, bei der das erste Rollbauteil (17) auf einer Rollfläche (21) an dem zweiten Rollbauteil (18) rollt.

2. Nadelmodul (4; 4a) nach Anspruch 1, **dadurch gekennzeichnet, dass** die Rolleinrichtung (16) ein drittes Rollbauteil (19) aufweist und dass beim Übertragen der Antriebsbewegung eine weitere Rollbewegung ausgeführt wird, bei der das erste Rollbauteil (17) auf einer weiteren Rollfläche (22) an dem dritten Rollbauteil (19) abrollt.

3. Nadelmodul (4; 4a) nach Anspruch 2, **dadurch gekennzeichnet, dass** bei der Rolleinrichtung eine der folgenden Ausführungen vorgesehen ist:
- das zweite und das dritte Rollbauteil (18, 19) sind in Bezug auf die Drehachsel (20) des ersten Rollbauteils (17) auf gegenüberliegenden Seiten angeordnet; und
- das zweite und das dritte Rollbauteil (18, 19) sind in Bezug auf die Drehachsel (20) des ersten Rollbauteils (17) auf der gleichen Seite benachbart zueinander angeordnet.

4. Nadelmodul (4; 4a) nach Anspruch 2 oder 3, **dadurch gekennzeichnet, dass** bei der Rolleinrichtung eine der folgenden Ausführungen vorgesehen ist:
- das erste Rollbauteil (17) ist mit einem ersten Radbauteil gebildet, wobei die Rolleinrichtung (16) betreibbar ist, dass das erste Radbauteil bei der Rollbewegung auf der Rollfläche (21) des zweiten Rollbauteils (18) und bei der weiteren Rollbewegung auf der weiteren Rollfläche (22) des dritten Rollbauteils (19) abrollt; und
- das erste Rollbauteil (17) ist mit einem ersten und einem zweiten Radbauteil gebildet, die jeweils auf der Drehachsel (20) drehbar gelagert sind, wobei das erste und das zweite Radbauteil unterschiedliche Raddurchmesser aufweisen und die Rolleinrichtung (16) betreibbar ist, dass das erste Radbauteil bei der Rollbewegung auf der Rollfläche (21) des zweiten Rollbauteils (18) und das zweite Radbauteil bei der weiteren Rollbewegung auf der weiteren Rollfläche (22) des dritten Rollbauteils (19) abrollt.

5. Nadelmodul (4; 4a) nach Anspruch 4, **dadurch gekennzeichnet, dass** das erste Radbauteil einen kleineren Raddurchmesser als das zweite Radbauteil aufweist.

6. Nadelmodul (4; 4a) nach mindestens einem der vorangehenden Ansprüche, **dadurch gekennzeichnet, dass** bei der Rolleinrichtung (16) zumindest eine der folgenden Ausführungen vorgesehen ist:
- die Rollfläche (21) an dem zweiten Rollbauteil (18) ist eine ebene Rollfläche; und
- die weitere Rollfläche (22) an dem dritten Rollbauteil (19) ist eine weitere ebene Rollfläche,

7. Nadelmodul (4; 4a) nach mindestens einem der vorangehenden Ansprüche, **dadurch gekennzeichnet, dass** bei der Rolleinrichtung (16) eine der folgenden Ausführungen vorgesehen ist:
- bei der Rollbewegung verbleibt eine Drehachsel (20) des ersten Rollbauteils (17) in einer ortfesten Achsenlage im Modulgehäuse (5), während sich das zweite Rollbauteil (18) mit der hieran gebildeten Rollfläche (21) relativ zum Modulgehäuse (5) verlagert, wobei das zweite Rollbauteil (18) zum abtriebsseitigen Einkoppeln der übertragenen Antriebsbewegung mit dem Nadelschaft (14) verbunden ist; und
- bei der Rollbewegung verbleibt das dritte Rollbauteil (19) mit der hieran gebildeten weiteren Rollfläche (22) in einer ortfesten Bauteillage im Modulgehäuse (5), während sich das erste Rollbauteil (17) mit seiner Drehachsel (20) und das zweite Rollbauteil (18) mit seiner Rollfläche (21) relativ zum Modulgehäuse (5) verlagern, wobei das erste und / oder das zweite Rollbauteil (17, 18) zum abtriebsseitigen Einkoppeln der übertragenen Antriebsbewegung mit dem Nadelschaft (14) verbunden sind.

8. Nadelmodul (4; 4a) nach den Ansprüchen 2 und 7, dadurch g e **kennzeichnet**, dass die Rolleinrichtung (16) betreibbar ist, das dritte Rollbauteil (19) mit der hieran gebildeten weiteren Rollfläche (22) relativ zum Modulgehäuse (5) zu verlagern, wenn bei der Rollbewegung die Drehachsel (20) des ersten Rollbauteils (17) in der ortfesten Achsenlage im Modulgehäuse (5) verbleibt, wobei das zweite und das dritte Rollbauteil (18, 19) hierbei in entgegengesetzte Bewegungsrichtungen verlagerbar sind.

9. Nadelmodul (4; 4a) nach mindestens einem der vorangehenden Ansprüche, **dadurch gekennzeichnet, dass** die Rolleinrichtung (16) betreibbar ist, das zweite und / oder das dritte Rollbauteil (18, 19) bei der Rollbewegung / der weiteren Rollbewegung zum Verlagern in dem Modulgehäuse (5) linear zu bewegen.

10. Nadelmodul (4; 4a) nach mindestens einem der vorangehenden Ansprüche, **dadurch gekennzeichnet, dass** bei der Rolleinrichtung (16) eine der folgenden Ausführungen vorgesehen ist:
- mit dem ersten Rollbauteil (17) ist ein antriebsseitiges Rollbauteil gebildet, auf welches die Antriebsbewegung einkoppelbar ist, und mit dem zweiten oder dem dritten Rollbauteil (18, 19) ist ein abtriebsseitiges Rollbauteil gebildet, welches betreibbar ist, die übertragene Antriebsbewegung abtriebsseitig auf den Nadelschaft (14) einzukoppeln; und
- mit dem zweiten oder dem dritten Rollbauteil (18, 19) ist ein antriebsseitiges Rollbauteil gebildet, auf welches die Antriebsbewegung einkoppelbar ist, und mit dem ersten Rollbauteil (17) ist ein abtriebsseitiges Rollbauteil gebildet, welches betreibbar ist, die übertragene Antriebsbewegung abtriebsseitig auf den Nadelschaft (14) einzukoppeln.

11. Nadelmodul (4; 4a) nach mindestens einem der vorangehenden Ansprüche, **dadurch gekennzeichnet, dass** bei der Rolleinrichtung (16) mindestens eine der folgenden Ausführungen vorgesehen ist:
- zwischen dem ersten und dem zweiten Rollbauteil (17, 18) ist eine Reibschlussverbindung ausgebildet und
- zwischen dem ersten und dem dritten Rollbauteil (17, 19) ist eine Reibschlussverbindung ausgebildet.

12. Nadelmodul (4; 4a) nach mindestens einem der vorangehenden Ansprüche, **dadurch gekennzeichnet, dass** bei der Rolleinrichtung (16) mindestens eine der folgenden Ausführungen vorgesehen ist:
- zwischen dem ersten und dem zweiten Rollbauteil (17, 18) ist eine Formschlussverbindung ausgebildet und
- zwischen dem ersten und dem dritten Rollbauteil (17, 19) ist eine Formschlussverbindung ausgebildet.

13. Nadelmodul (4; 4a) nach mindestens einem der vorangehenden Ansprüche, **dadurch gekennzeichnet, dass** die Rolleinrichtung (16) ist betreibbar, die Rollbewegung und / oder die weitere Rollbewegung als reines Rollen auszuführen.

14. Nadelmodul (4; 4a) nach mindestens einem der vorangehenden Ansprüche, **dadurch gekennzeichnet, dass** die Übertragungseinrichtung eingerichtet ist, eine lineare repetierende Antriebsbewegung mit der Wiederholfrequenz zu übertragen.

15. Handgerät (1) zum lokalen Aufstechen einer menschlichen oder einer tierischen Haut, mit einem Nadelmodul (4; 4a) nach mindestens einem der vorangehenden Ansprüche und einer Antriebseinrichtung (3a), die zum Übertragen einer von der Antriebseinrichtung (3a) bereitgestellten Antriebsbewegung auf das Nadelmodul (4; 4a) funktional an das Nadelmodul (4; 4a) koppelt.

16. Verfahren zum Betreiben eines Nadelmoduls (4; 4a), mit
- Bereitstellen eines Nadelmoduls (4; 4a), aufweisend:
- ein Modulgehäuse (5), welches eine Modulspitze und eine im Bereich der Modulspitze ausgebildete frontseitige Modulöffnung (7) aufweist;
- eine Stecheinrichtung, welche wenigstens eine an einem Nadelschaft (14) angeordnete Stechnadel (9) mit einer Nadelspitze (10) aufweist und in dem Modulgehäuse (5) aufgenommen ist; und
- eine Übertragungseinrichtung, die wenigstens teilweise in dem Modulgehäuse (5) aufgenommen ist und eine Rolleinrichtung (16) aufweist;
- antriebsseitiges Einleiten einer mit einer Wiederholfrequenz bereitgestellte Antriebsbewegung auf die Übertragungseinrichtung;
- Übertragen der Antriebsbewegung mittels der Übertragungseinrichtung, wobei hierbei mittels der Rolleinrichtung (16) eine Rollbewegung ausgeführt wird, bei der ein erstes Rollbauteil (17) der Rolleinrichtung (16), welches drehbar gelagert ist, auf einer Rollfläche (21) an einem zweiten Rollbauteil (18) der Rolleinrichtung (16) abrollt; und
- abtriebsseitiges Einkoppeln der übertragenen Antriebsbewegung auf den Nadelschaft (14), derart, dass die wenigstens eine Stechnadel (9) mittels einer linearen Bewegung wiederholt zwischen einer eingefahrenen und einer ausgefahrenen Stellung verlagert wird, wobei sich die wenigstens eine Stechnadel (9) zumindest in der ausgefahrenen Stellung durch die fronseitige Modulöffnung (7) erstreckt und die Nadelspitze hierbei außerhalb des Modulgehäuses (5) angeordnet ist.

## Geänderte Patentansprüche

### Geänderte Patentansprüche gemäss Regel 137(2) EPÜ.

1. Handgerät (1) zum lokalen Aufstechen einer menschlichen oder einer tierischen Haut, mit
- einem Nadelmodul (4; 4a) und
- einer Antriebseinrichtung (3a), die zum Übertragen einer von der Antriebseinrichtung (3a) bereitgestellten, linearen repetierenden Antriebsbewegung auf das Nadelmodul (4; 4a) funktional an das Nadelmodul (4; 4a) koppelt;
wobei das Nadelmodul (4; 4a) Folgendes aufweist:
- ein Modulgehäuse (5), welches eine Modulspitze und eine im Bereich der Modulspitze ausgebildete frontseitige Modulöffnung (7) aufweist;
- eine Stecheinrichtung, welche wenigstens eine an einem Nadelschaft (14) angeordnete Stechnadel (9) mit einer Nadelspitze (10) aufweist und in dem Modulgehäuse (5) aufgenommen ist, derart, dass die wenigstens eine Stechnadel (9) mittels einer linearen Bewegung zwischen einer eingefahrenen und einer ausgefahrenen Stellung verlagerbar ist, wobei sich die wenigstens eine Stechnadel (9) zumindest in der ausgefahrenen Stellung durch die fronseitige Modulöffnung (7) erstreckt und die Nadelspitze hierbei außerhalb des Modulgehäuses (5) angeordnet ist; und
- eine Übertragungseinrichtung, die
- wenigstens teilweise in dem Modulgehäuse (5) aufgenommen und betreibbar ist, die antriebsseitig mit einer Wiederholfrequenz bereitgestellte Antriebsbewegung auf den Nadelschaft (14) zu übertragen, derart, dass die übertragene Antriebsbewegung abtriebsseitig auf die wenigstens eine Stechnadel (9) einkoppelbar ist, um diese wiederholt zwischen der eingefahrenen und der ausgefahrenen Stellung zu verlagern; und
- eine Rolleinrichtung (16) aufweist, die ein erstes Rollbauteil (17), welches drehbar gelagert ist, ein zweites Rollbauteil (18) und ein drittes Rollbauteil (19) aufweist, wobei die Antriebsbewegung auf die Rolleinrichtung (16) einleitbar ist, derart, dass beim Übertragen der Antriebsbewegung eine Rollbewegung, bei der das erste Rollbauteil (17) auf einer Rollfläche (21) an dem zweiten Rollbauteil (18) rollt, und eine weitere Rollbewegung ausgeführt werden, bei der das erste Rollbauteil (17) auf einer weiteren Rollfläche (22) an dem dritten Rollbauteil (19) rollt.

2. Handgerät (1) nach Anspruch 1, **dadurch gekennzeichnet, dass** bei der Rolleinrichtung eine der folgenden Ausführungen vorgesehen ist:
- das zweite und das dritte Rollbauteil (18, 19) sind in Bezug auf die Drehachse (20) des ersten Rollbauteils (17) auf gegenüberliegenden Seiten angeordnet; und
- das zweite und das dritte Rollbauteil (18, 19) sind in Bezug auf die Drehachse (20) des ersten Rollbauteils (17) auf der gleichen Seite benachbart zueinander angeordnet.

3. Handgerät (1) nach mindestens einem der vorangehenden Ansprüche, dadurch **ge** - **k e n n z e i c h n e t,** dass bei der Rolleinrichtung eine der folgenden Ausführungen vorgesehen ist:
- das erste Rollbauteil (17) ist mit einem ersten Radbauteil gebildet, wobei die Rolleinrichtung (16) betreibbar ist, dass das erste Radbauteil bei der Rollbewegung auf der Rollfläche (21) des zweiten Rollbauteils (18) und bei der weiteren Rollbewegung auf der weiteren Rollfläche (22) des dritten Rollbauteils (19) abrollt; und
- das erste Rollbauteil (17) ist mit einem ersten und einem zweiten Radbauteil gebildet, die jeweils auf der Drehachse (20) drehbar gelagert sind, wobei das erste und das zweite Radbauteil unterschiedliche Raddurchmesser aufweisen und die Rolleinrichtung (16) betreibbar ist, dass das erste Radbauteil bei der Rollbewegung auf der Rollfläche (21) des zweiten Rollbauteils (18) und das zweite Radbauteil bei der weiteren Rollbewegung auf der weiteren Rollfläche (22) des dritten Rollbauteils (19) abrollt.

4. Handgerät (1) nach Anspruch 3, **dadurch gekennzeichnet, dass** das erste Radbauteil einen kleineren Raddurchmesser als das zweite Radbauteil aufweist.

5. Handgerät (1) nach mindestens einem der vorangehenden Ansprüche, dadurch **ge- kennzeichnet**, dass bei der Rolleinrichtung (16) zumindest eine der folgenden Ausführungen vorgesehen ist:
- die Rollfläche (21) an dem zweiten Rollbauteil (18) ist eine ebene Rollfläche; und
- die weitere Rollfläche (22) an dem dritten Rollbauteil (19) ist eine weitere ebene Rollfläche,

6. Handgerät (1) nach mindestens einem der vorangehenden Ansprüche, dadurch **ge- kennzeichnet**, dass bei der Rolleinrichtung (16) eine der folgenden Ausführungen vorgesehen ist:
- bei der Rollbewegung verbleibt eine Drehachse (20) des ersten Rollbauteils (17) in einer ortfesten Achsenlage im Modulgehäuse (5), während sich das zweite Rollbauteil (18) mit der hieran gebildeten Rollfläche (21) relativ zum Modulgehäuse (5) verlagert, wobei das zweite Rollbauteil (18) zum abtriebsseitigen Einkoppeln der übertragenen Antriebsbewegung mit dem Nadelschaft (14) verbunden ist; und
- bei der Rollbewegung verbleibt das dritte Rollbauteil (19) mit der hieran gebildeten weiteren Rollfläche (22) in einer ortfesten Bauteillage im Modulgehäuse (5), während sich das erste Rollbauteil (17) mit seiner Drehachse (20) und das zweite Rollbauteil (18) mit seiner Rollfläche (21) relativ zum Modulgehäuse (5) verlagern, wobei das erste und / oder das zweite Rollbauteil (17, 18) zum abtriebsseitigen Einkoppeln der übertragenen Antriebsbewegung mit dem Nadelschaft (14) verbunden sind.

7. Handgerät (1) nach Anspruch 6, dadurch **gek e n n z e i c h n e t,** dass die Rolleinrichtung (16) betreibbar ist, das dritte Rollbauteil (19) mit der hieran gebildeten weiteren Rollfläche (22) relativ zum Modulgehäuse (5) zu verlagern, wenn bei der Rollbewegung die Drehachse (20) des ersten Rollbauteils (17) in der ortfesten Achsenlage im Modulgehäuse (5) verbleibt, wobei das zweite und das dritte Rollbauteil (18, 19) hierbei in entgegengesetzte Bewegungsrichtungen verlagerbar sind.

8. Handgerät (1) nach mindestens einem der vorangehenden Ansprüche, dadurch **ge- kennzeichne**t, dass die Rolleinrichtung (16) betreibbar ist, das zweite und / oder das dritte Rollbauteil (18, 19) bei der Rollbewegung / der weiteren Rollbewegung zum Verlagern in dem Modulgehäuse (5) linear zu bewegen.

9. Handgerät (1) nach mindestens einem der vorangehenden Ansprüche, dadurch **ge- kennzeichnet**, dass bei der Rolleinrichtung (16) eine der folgenden Ausführungen vorgesehen ist:
- mit dem ersten Rollbauteil (17) ist ein antriebsseitiges Rollbauteil gebildet, auf welches die Antriebsbewegung einkoppelbar ist, und mit dem zweiten oder dem dritten Rollbauteil (18, 19) ist ein abtriebsseitiges Rollbauteil gebildet, welches betreibbar ist, die übertragene Antriebsbewegung abtriebsseitig auf den Nadelschaft (14) einzukoppeln; und
- mit dem zweiten oder dem dritten Rollbauteil (18, 19) ist ein antriebsseitiges Rollbauteil gebildet, auf welches die Antriebsbewegung einkoppelbar ist, und mit dem ersten Rollbauteil (17) ist ein abtriebsseitiges Rollbauteil gebildet, welches betreibbar ist, die übertragene Antriebsbewegung abtriebsseitig auf den Nadelschaft (14) einzukoppeln.

10. Handgerät (1) nach mindestens einem der vorangehenden Ansprüche, dadurch **ge- kennzeichnet**, dass bei der Rolleinrichtung (16) mindestens eine der folgenden Ausführungen vorgesehen ist:
- zwischen dem ersten und dem zweiten Rollbauteil (17, 18) ist eine Reibschlussverbindung ausgebildet und
- zwischen dem ersten und dem dritten Rollbauteil (17, 19) ist eine Reibschlussverbindung ausgebildet.

11. Handgerät (1) nach mindestens einem der vorangehenden Ansprüche, dadurch **ge- kennzeichnet**, dass bei der Rolleinrichtung (16) mindestens eine der folgenden Ausführungen vorgesehen ist:
- zwischen dem ersten und dem zweiten Rollbauteil (17, 18) ist eine Formschlussverbindung ausgebildet und
- zwischen dem ersten und dem dritten Rollbauteil (17, 19) ist eine Formschlussverbindung ausgebildet.

12. Handgerät (1) nach mindestens einem der vorangehenden Ansprüche, dadurch **ge- kennzeichnet,** dass die Rolleinrichtung (16) ist betreibbar, die Rollbewegung und / oder die weitere Rollbewegung als reines Rollen auszuführen.

13. Verfahren zum Betreiben eines Handgeräts (1), mit
- Bereitstellen eines Handgeräts (1) mit einem Nadelmodul (4; 4a) und einer Antriebseinrichtung (3a), die zum Übertragen einer von der Antriebseinrichtung (3a) bereitgestellten, linearen repetierenden Antriebsbewegung auf das Nadelmodul (4; 4a) funktional an das Nadelmodul (4; 4a) koppelt, wobei das Nadelmodul (4; 4a) Folgendes aufweist:
- ein Modulgehäuse (5), welches eine Modulspitze und eine im Bereich der Modulspitze ausgebildete frontseitige Modulöffnung (7) aufweist;
- eine Stecheinrichtung, welche wenigstens eine an einem Nadelschaft (14) angeordnete Stechnadel (9) mit einer Nadelspitze (10) aufweist und in dem Modulgehäuse (5) aufgenommen ist; und
- eine Übertragungseinrichtung, die wenigstens teilweise in dem Modulgehäuse (5) aufgenommen ist und eine Rolleinrichtung (16) aufweist;
- antriebsseitiges Einleiten der von der Antriebseinrichtung (3a) mit einer Wiederholfrequenz bereitgestellten Antriebsbewegung auf die Übertragungseinrichtung;
- Übertragen der Antriebsbewegung mittels der Übertragungseinrichtung, wobei hierbei mittels der Rolleinrichtung (16)
- eine Rollbewegung ausgeführt wird, bei der ein erstes Rollbauteil (17) der Rolleinrichtung (16), welches drehbar gelagert ist, auf einer Rollfläche (21) an einem zweiten Rollbauteil (18) der Rolleinrichtung (16) rollt, und
- eine weitere Rollbewegung ausgeführt wird, bei der das erste Rollbauteil (17) auf einer weiteren Rollfläche (22) an dem dritten Rollbauteil (19) rollt; und
- abtriebsseitiges Einkoppeln der übertragenen Antriebsbewegung auf den Nadelschaft (14), derart, dass die wenigstens eine Stechnadel (9) mittels einer linearen Bewegung wiederholt zwischen einer eingefahrenen und einer ausgefahrenen Stellung verlagert wird, wobei sich die wenigstens eine Stechnadel (9) zumindest in der ausgefahrenen Stellung durch die fronseitige Modulöffnung (7) erstreckt und die Nadelspitze hierbei außerhalb des Modulgehäuses (5) angeordnet ist.
